# EUROPEAN PATENT APPLICATION

(11) **EP 3 130 294 A1**
(43) Date of publication of application: **15.02.2017**
(21) Application number: 16181937.0
(22) Date of filing: 29.07.2016
(51) Int. Cl.: A61B 17/115

(54) **DISPOSABLE CONNECTING UNIT OF A CIRCULAR STAPLER AND A CIRCULAR STAPLER**

(30) Priority: 31.07.2015 PL 41336315
(71) Applicant: Grena Limited, Brentford TW8 9HH (GB)
(72) Inventor: Brodaczewski, Wieslaw, 05-410 Józefów (PL); Decewicz, Andrzej, 05-410 Józefów (PL); Redosz, Radoslaw, 02-785 Warsaw (PL)
(74) Representative: Kondrat, Mariusz

(57) **Abstract**

The object of the invention is a disposable connecting unit of a circular stapler comprising a load, which is mounted on a body with a load nut constituting the proximal end of the outer load sleeve connected to the load housing comprising in the upper part a ring with a circular knife supported on the load impactor and an anvil, wherein the anvil is concentrically placed on the needle passing through the inner load sleeve, characterised in that on the outer surface of the inner sleeve there is positioned a load spring withdrawing the circular knife, which extends between the distal end of the load impactor and a flange of the inner load sleeve. The invention also relates to a circular stapler for connecting tissue fragments, comprising a shared disposable connecting unit.

## Description

The invention relates to a disposable connecting unit of a circular stapler used in surgical anastomosis, especially intended for intestinal connections, wherein the connection is done using surgical staples. The invention also relates to a circular stapler comprising a disposable connecting unit.

EP2524654A2 discloses a circular stapler for stapling, which comprises a drive shaft, a ejector strap and a friction reduction means. The drive shaft is designed to adjust the distance between the anvil system and the stapling head system. The ejector strap is designed to launch and activate the stapling head system to release staples. The ejector strap forms the first contact surface, which faces the corresponding second contact surface supplied on the drive shaft.

EP1836974A2 describes an assembly for connecting tissues comprising an anvil assembly and a body positioned opposite each other along the shaft and arranged so as to be close to each other and an applicator mounted on the anvil system and configured in order to hold in it a material for wound treatment and for dispensing from it a material for the treatment of wounds. The applicator may comprise a sleeve/tunnel functionally connected to a retaining means, wherein the material for the treatment of wounds is retained inside and dispensed from the sleeve. The applicator may further comprise an ampoule with its position selectively fixed inside the sleeve, wherein the wound treatment material is retained inside and dispensed from the ampoule. Document EP2163211A3 discloses a surgical stapling device comprising an elongated body with a front and a rear portion, and a head portion, which is placed in the proximity of the rear portion of the body. The front portion comprises a cover assembly and a retaining means, which is movable relative to the cover assembly between the near and the remote position. The anvil assembly comprises a rod/a central shaft and an anvil head. The central shaft comprises at least one orifice transversely crossing the central shaft, which is configured and dimensioned in order to obtain a flexible means, thanks to which the flexible means extends transversely across the central shaft.

Description in EP2649949 discloses a surgical stapler comprising: a tubular body; a container assembly placed at the distal end of the body to release an annular series of staples, and an anvil means placed at the distal end of the tubular body and placed opposite a cartridge assembly clenching the staple in the tissue following the ejection of the staple from the cartridge assembly. An anvil assembly having a suitable annular staple system, a simple configuration piston, wherein the staples are clenched by the pistons so that the arms extend to or beyond the rear span. Application EP2730238 A1 describes a surgical device for stapling, which consists of blocking the reciprocation of the anvil assembly to the cartridge assembly following the initial firing of a surgical stapling device. The surgical device for stapling comprises a blocking mechanism, for example a blocking sleeve, which is adapted to connect the retaining means assembly and the cartridge assembly following the firing to block the reciprocation of the retaining means assembly to the cartridge/capsule assembly.

A circular device for stapling disclosed in application EP2769687A1 comprises a grip assembly, an elongated body, which extends from the grip assembly, a cartridge assembly mounted at the distal end of the elongated body, a means supporting a roller container, a element of a setter ring and a cutting assembly.

The cartridge assembly determines an annular limiting gap and comprises a housing, an impactor means and a body of the container. The housing and a connector of the body of the cartridge together form a gap between them. The impactor means is supported inside the housing and may move between a first position and a second position. The connector of the body of the cartridge is supported inside the housing and has a tissue engaging surface, which extends on the annular edge.

Document US2015/0083772 A1 discloses a surgical circular stapler, which has a body, a shaft, a stapling assembly, an motor, a cam assembly, and a firing assembly. Shaft/spindle extends distally from the body. The stapling assembly is attached to the distal end of the spindle. Longitudinal transposition of the firing assembly causes the stapling assembly to drive multiple staples in a roller system to provide two clearances of the tissue together. The stapling assembly may further lead the blade to sever the excess of the tissue inside the circular system of the staples. The motor is adapted to rotate the cam assembly. Rotation of the cam assembly results in a longitudinal transposition of the firing assembly. A single rotation of the cam assembly is adapted to drive the firing assembly from the proximal position to the distal position and again back to the proximal position.

The surgical circular stapler described in application US2015/0083775 has a grip assembly, a shaft, a stapling assembly, a motor, a driving assembly and a firing assembly. The shaft extends distally from the grip. The stapling assembly is attached to the distal end of the spindle. Longitudinal transposition of the firing assembly causes the stapling assembly to drive multiple staples of the circular system. The motor is adapted to rotate the drive assembly, thereby gearing the firing assembly. The resilient means presses on the firing assembly in the proximal direction. Thanks to the cooperation between the firing assembly and the resilient means, the firing assembly is driven in the distal and proximal direction to end a firing stroke in response to a rotation of the drive assembly with a single rotation.

Known circular staplers, within the connecting unit (the connecting head) comprise a knife, which is used to remove excess tissue remaining following the connection of intestines. Unfortunately, these staplers have no ability to withdraw the knife after performing the connection of intestines. In the known staplers, the knife remains in the extended position and when removing the stapler from the connection site, it causes uncontrolled tearing of the tissue of the intestine, which leads to bleeding, and even non-tightness of the connection.

The aim of the invention was to develop such a structure of a disposable connecting unit of a circular stapler, that would allow for withdrawing the impactor together with the knife. Such a solution would prevent damaging the tissue to be connected.

In the solution according to the invention, a spring load was used located on the inner sleeve of the load, supported on the lower surface of the load impactor, wherein the inner load sleeve has in its upper part a flange, which retains the spring on the inner load sleeve and prevents it from coming out. When performing the connection of tissues, when the load impactor initiates the formation of staples, the spring load is tightened, which, after performing the connection of tissues, causes the load impactor to withdraw to the initial position. After the withdrawal of the load impactor, when it is in the initial position, the knife is withdrawn, which consequently prevents the tissue from being damaged, and this favourable affects the quality of the connection. The essence of the invention is a disposable connecting unit of a circular stapler comprising a load, which is mounted on a body with a load nut constituting the proximal end of the outer load sleeve connected to the load housing comprising in the upper part a ring with a circular knife supported on the load impactor and an anvil, wherein the anvil is concentrically placed on a needle passing through the inner load sleeve, characterised in that on the outer surface of the inner sleeve there is positioned a load spring withdrawing the circular knife, which extends between the distal end of the load impactor and a flange of the inner load sleeve.

The invention also relates to a circular stapler for connecting tissue fragments, comprising a shared connecting unit placed on a body, comprising a load connected to an anvil by means of a tendon system; a grip, an indicator, a knob and an impactor system characterised in that the connecting unit is constituted by a disposable connecting unit described above.

The disposable connecting unit was provided with an outer load sleeve. This facilitates to mount the load on the stapler and ensures a stable fastening of the load on the stapler.

The circular stapler is used in general surgery, thoracic surgery, bariatric surgery, in colon and rectum resection, for performing end-to-end, side-to-end and side-to-side connections throughout the gastrointestinal tract. Before the first use, the stapler should be washed from the residue of the preservative and sterilised in an autoclave. After each use, the stapler needs to be washed and sterilised, so that it can be used again. This applies only to the device itself, as the loads and the anvil are delivered in a sterile condition and are intended for single use. The sterile load needs to be screwed clockwise on the distal portion of the stapler. Preparing the stapler for use requires applying purse-string sutures at both ends to be connected. Then, using the knob clockwise, it is necessary to completely hide the needle in the body of the device. The anvil is inserted into the light of the organ and the purse string suture is tightened on the opening of the purse-string suture. Then, the circular stapler is inserted with the anvil disconnected into the second portion of the organ to be connected until the light is occluded. By rotating the knob counter-clockwise, it is necessary to completely remove the needle piercing the occluded light of the organ. In the next step, the tissue slides to the base of the needle exposing the suture ligature site. Then, the spindle of the anvil needs to be connected to the needle. For a proper connection, an audible click is required. Then, a space (a gap) is determined between the anvil and the load by rotating the knob clockwise. When the indicator shows the appropriate value (1 mm), the grip is unblocked. The stapler is ready for use. To perform the connection, the grip is pressed, in a strong and steady movement. Then, the grip needs to be released, and it returns to the initial position. Rotating the knob counter-clockwise by no more than 2 to 3 rotations will lead to distancing the anvil from the load. The stapler needs to be removed by gently pulling backwards in a swinging movement. After removing the disposable connecting unit it is necessary to check the integrity of the connection, while the tissue resected by the circular knife needs to be carefully verified whether it forms a full ring and whether it comprises all the layers throughout the perimeter.

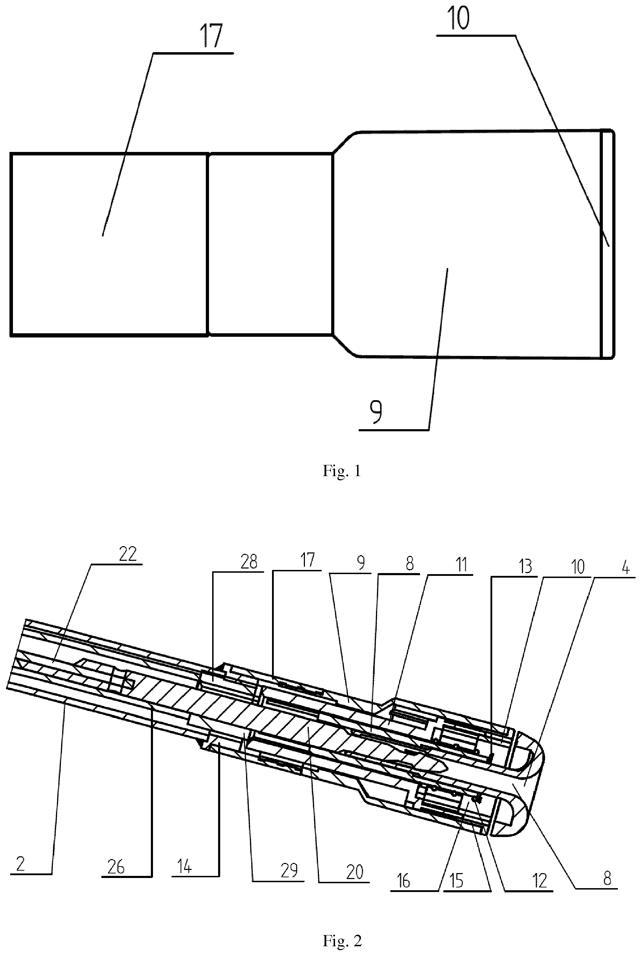

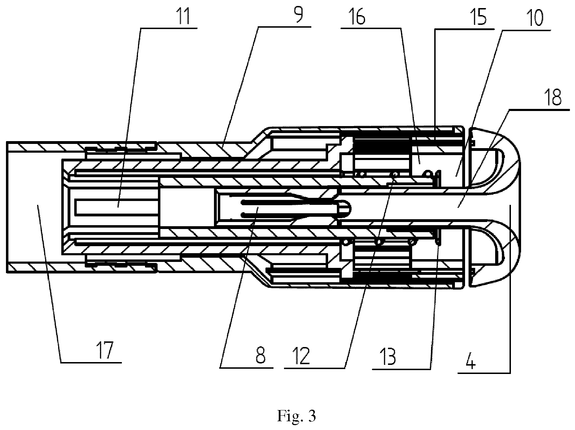

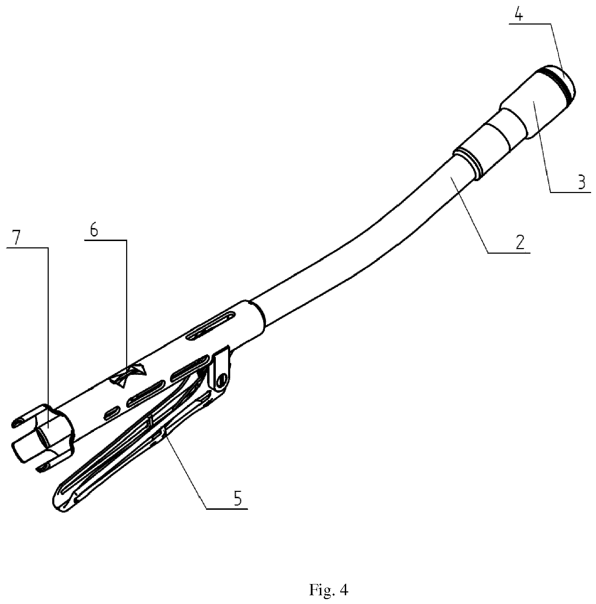

The object of the invention is shown in the embodiment and in the drawing, where fig. 1 shows a perspective view of the load, fig. 2 shows a cross section of the disposable connecting unit mounted on the body, fig. 3 shows a cross section of the disposable connecting unit, fig. 4 - a perspective view of the circular stapler.

The circular stapler comprises a shared disposable connecting unit 1 placed on the tendon system of the body 2. The disposable connecting unit 1 has a load 3 positioned opposite the anvil 4 placed on the needle 20. Inside the load 3 a ring 10 is placed comprising a set of surgical staples 15, situated opposite the anvil 4, slidably placed on the body 2 comprising a grip 5 and a tendon system 19 consisting of a needle 20, a tendon 22, a tendon support 21, a bolt 23 and a lead screw 24 ended with a knob 7, wherein the tendon system is positioned inside the body 2.

The circular stapler inside the body 2 also comprises an impactor system 25 consisting of an impactor tube 26, a proximal means of the impactor 27 and a distal means of the impactor 28, wherein the distal means of the impactor 28 is formed cylindrically and is fitted to the inner surface of the body 2.

The disposable connecting unit comprises a load 3 and an anvil 4 placed on the tendon system 19, consisting of a needle 20, a tendon 22, a tendon support 21, a bolt 23 and a lead screw 24. The anvil 4 is movably connected to the load 3 by means of an anvil sleeve 18 clippingly mounted on the needle 20. The needle 20 constituting the distal end of the tendon system 19 passes concentrically through the inner load sleeve 8.

The load 3 in the form of a sleeve is composed of a load housing 9, an outer load sleeve 17, a load nut 14, an inner load sleeve 8, a ring 10, a circular knife 16 and a load impactor 11, and inside the ring 10 a circular knife 16 is placed supported on the the lower surface of the load impactor 11. On the outer side of the inner load sleeve 8 between the upper surface of the load impactor 11 and a flange 13, a load spring 12 is placed. Through the inner load sleeve 8 the needle 20 passes on which an anvil is lockably mounted 4.

The ring 10 comprises a set of staples in a number between 18 and 32 depending on the size of the load.

After fastening the load 3 with the nut load 14 on the body 2 and of the anvil 3, rotating the knob 7 causes the anvil 4 to approach the load 3 in a disposable connecting unit 1. The knob 7 causes the tendon system 19 to slide, and together with it the anvil 4 mounted on the needle 20. Once the appropriate size of the gap between the anvil 4 and the load 3 amounting to between 1 and 1.1 mm, the grip 5 is unblocked, and it is possible to perform the connection by pressing it. Pressing the grip 5 located in the proximal means of the impactor 27 causes the impactor tube 26 and the distal means of the impactor 28 to slide. The distal means of the impactor 28 then pushes the load impactor 11 located in the load housing 9. The load impactor 11 causes the ejection of the surgical staples 15 located in the ring 10 and of the circular knife 16. The surgical staples 15 are formed on the recesses located in the anvil 4 during the execution of the connection. After releasing the grip 5, the load impactor 11 returns to the entry site thanks to the spring 12 placed between the inner load sleeve 8 and the load impactor 11. The withdrawal of the load impactor 11 also causes the withdrawal of the circular knife 16.

## Claims

1. A disposable connecting unit of a circular stapler comprising a load (3), which is mounted on a body (2) with a load nut (14) constituting the proximal end of the outer load sleeve (17) connected to the load housing (9) comprising in the upper part a ring (10) with a circular knife (16) supported on the load impactor (11) and an anvil (4), wherein the anvil (4) is concentrically placed on the needle (20) passing through the inner load sleeve (8), **characterised in that** on the outer surface of the inner sleeve (8) there is positioned a load spring (12) withdrawing the circular knife (16), which extends between the distal end of the load impactor (11) and a flange (13) of the inner load sleeve (8).

2. A circular stapler for connecting tissue fragments, comprising a shared connecting unit placed on a body comprising a load connected to an anvil by means of a tendon system; a grip, an indicator, a knob and an impactor system **characterised in that** the connecting unit is constituted by a disposable connecting unit described in claim 1.
